Europäisches Patentamt

European Patent Office

Office européen des brevets

(19)

(11) Veröffentlichungsnummer: **0 098 413**
**B1**

(12) # EUROPÄISCHE PATENTSCHRIFT

(45) Veröffentlichungstag der Patentschrift:
13.01.88

(51) Int. Cl.⁴: **A 61 B 5/04,** A 61 B 5/05,
A 61 H 39/00, G 01 R 29/24

(21) Anmeldenummer: 83105771.6

(22) Anmeldetag: 13.06.83

(54) **Verfahren und Vorrichtung für das kontaktlose Messen von Ladungskonzentrationen und Potentialunterschieden bei biologischen Organismen.**

(30) Priorität: 01.07.82 LU 84250

(43) Veröffentlichungstag der Anmeldung:
18.01.84 Patentblatt 84/3

(45) Bekanntmachung des Hinweises auf die Patenterteilung:
13.01.88 Patentblatt 88/2

(84) Benannte Vertragsstaaten:
AT BE CH DE FR GB IT LI LU NL SE

(56) Entgegenhaltungen:
CH-A-352 781
DE-A-2 520 033
DE-A-2 928 826
DE-B-1 281 573
FR-A-2 329 258
US-A-3 611 127
US-A-3 774 108
US-A-4 205 267

XEROX DISCLOSURE JOURNAL, Band 2, Nr. 6, November/Dezember 1977, STAMFORD/CONN. (US), R. CASE: "Liquid Cristal Electrometer", Seite 91

Die Akte enthält technische Angaben, die nach dem Eingang der Anmeldung eingereicht wurden und die nicht in dieser Patentschrift enthalten sind.

(73) Patentinhaber: **MARDICE HOLDING S.à.r.l., 46 rue du Cimetière, L-1338 Luxemburg (LU)**

(72) Erfinder: **Der Erfinder hat auf seine Nennung verzichtet**

(74) Vertreter: **Meyers, Ernest, Office de Brevets Freylinger & Associés 46 rue du Cimetière B.P. 1153, L-1011 Luxembourg (LU)**

## Beschreibung

Die Erfindung betrifft eine Vorrichtung für das kontaktlose Messen von Ladungskonzentrationen und Potentialunterschieden bei biologischen Organismen, insbesondere bei Menschen, Tieren und Pflanzen.

Es ist bekannt, dass unter gewissen Bedingungen elektrische Ladungskonzentrationen und Potentialunterschiede in Organismen bestehen und entstehen. Pathologische Prozesse, Genesungsprozesse und Informationsprozesse verlaufen nach einem elektrischen Gleichstromsystem in dem elektrische Ladungskonzentrationen und Potentialunterschiede zum Steuern der Genesungsprozesse und der Genesung selbst auftreten. Bei Pflanzen, Bäumen und Sträuchern ist es bekannt, dass die von der Sonne beschienenen Teile ein höheres elektrisches Potential aufweisen als die im Schatten befindlichen Teile.

Diese elektrischen Potentialunterschiede geben über die Lebensprozesse im Bioorganismus Auskunft. Weitere Einzelheiten ergeben sich aus " The basic biological data transmission and control system influenced by electrical forces" von R.O. Becker in "Annals of the New York Academy of Sciences", vol. 238, 11-10-1974, S. 236 - 241.

Um diese Potentialunterschiede zu messen kann man Geräte benützen, die elektrische Felder und ihre Grösse ausfindig machen. Es gibt mehrere dieser Geräte, die aber alle an der Bauart der Messonde scheitern und den Nachteil aufweisen, unpräzise zu sein.

Aus dem Artikel aus IEEE " Transactions on biomedical engineering (US)" Band BME-27, Nr 12, Dezember 1980, New York (US), B.C. Towe: "An air ionization biopotential electrode" Seiten 733-736 sind Messelektroden bekannt, die dazu dienen Oberflächenspannungsunterschiede kontaktlos zu messen. Die Anwendung solcher Elektroden fordert jedoch die Ionisation der zwischen der Organismusoberfläche und der Elektrode befindlichen Luft, um somit ein elektrisch leitendes Medium zu bilden. In diesem besonderen Fall wird die Ionisation durch eine auf der Elektrode befindliche ionisierende Strahlenquelle erreicht, insbesondere ein 100 μ Ci-radioaktives Polonium -210- Radioisotop, das X-Strahlen aussendet.

Aus der DE-A-2 928 828 sind eine Vorrichtung gemäß dem Obergriff des Anspruchs 1 sowie ein Verfahren zum kontaktlosen Messen von Ladungskonzentrationen und Potentialunterschieden bei biologischen Organismen bekannt. Gemäss diesem Verfahren wird eine Messelelektrode kontaktlos über die Organismusoberfläche gehalten, wobei das System Messelektrode/Organismusoberfläche einen Kondensator bildet. Es wird in dem Kondensator eine fortdauernde Änderung des elektrischen Feldes hervorgerufen und die dadurch entstehenden Ladungsverschiebungen werden gemessen und ausgewertet.

Das Ziel der Erfindung besteht darin eine Vorrichtung zu schaffen, die das kontaktlose Messen von Ladungskonzentrationen und elektrischen Potentialunterschieden bei Bio-Organismen ermöglicht. Diese neue Vorrichtung soll präzise arbeiten und weist dementsprechend neuartige Sonden auf.

Die Lösung der gestellten Aufgabe beruht auf einem bekannten Verfahren, bei dem in einem durch eine kontaktlos über die Organismusoberfläche gehaltene Messelelektrode und die Organismusoberfläche an sich gebildeten Kondensator eine fortdauernde Änderung des elektrostatischen Feldes hervorgerufen wird und die daraus resultierenden Ladungsverschiebungen gemessen und ausgewertet werden, die ein Ausdruck der im Bio-Organismus bestehenden Ladungskonzentrationen sind.

Gemäs diesem Messverfahren bildet die Sonde einen Kondensator mit der Oberfläche des Organismus. Beim Anschluss dieses Kondensators an eine äussere Schaltung fliesst ein elektrischer Strom, der die Ladung der Messelelektrode an diejenige der Organismusoberfläche anpasst. Eine Veränderung des elektrostatischen Feldes im Kondensator verursacht folglich eine entsprechende Veränderung der Ladung an der Messelektrode. Durch eine solche Veränderung fliesst also ein Strom durch die äussere Schaltung. Dieser Strom ist ein Mass für die Ladungsänderung und also auch den Potentialunterschied im Organismus.

Um dieses Verfahren anwenden zu können, wird, nach der vorliegenden Erfindung, eine Vorrichtung gemäss Patentanspruch 1 geschaffen.

Die oben genannten Schaltungen bestehen aus einer Ausgleichschaltung mit hochohmigem Widerstand, die dafür sorgt, dass die Ladungen an den beiden Kondensatorteilen immer entgegengesetzt bleiben und somit ein Ladungsverschub gemessen wird, sowie aus Filterstufen, Verstärkerstufen und Schaltungen zum Auswerten und Anzeigen der gemessene Werte.

Da die gemessenen Werte klein sind, ist auf eine besonders gute Abschirmung aller Bestandteile zu achten, um den äusseren Einfluss auf die sensible Vorrichtung so gering wie möglich zu halten. Deshalb muss die Elektrode besonders gut abgeschirmt werden, ebenso wie die Verbindungskabel und die Gehäuse. Das in der Abschirmung der Elektrode vorgesehene Fenster, durch das das elektrostatische Feld an die leitende Messelektrode heran kann wird zweckmässigerweise parallel zur Organismusoberfläche ausgerichtet.

Bevorzugte Ausführungsformen der Vorrichtung nach der Erfindung werden anhand der nachstehenden Zeichnungen näher erläutert:

Figur 1 zeigt eine schematische Anordnung der verschiedenen Bestandteile;

Figur 2 zeigt eine Ausführungsform des ersten Teils der Vorrichtung, die in Figur 1 schematisch dargestellt ist;

Figur 3 zeigt den zweiten Teil der in Figur 1 schematisch dargestellten Vorrichtung;

Figur 4 zeigt eine andere Ausführungsform des ersten Teils der in Figur 1 schematisch dargestellten Vorrichtung;

Figur 5 zeigt eine Anwendung der erfindungsgemässen Vorrichtung bei einem menschlichen Körperteil;

Figur 6 zeigt den Verlauf einer Kurve, die auf der Anzeige einer nicht gemäß Anspruch 1 ausgebildeten - jedoch ähnlichen - Vorrichtung erhalten wurde:

Figur 7 zeigt eine schematisch dargestellte Ausführungsform mit modulierbarer dielektrischer Konstante;

Figur 8 zeigt eine weitere Ausführungsform mit modulierbarer dielektrischer Konstante;

Figur 9 zeigt ein Blockschaltbild das zu den Ausführungsformen gemäss den Figuren 7 oder 8 passt.

In den Figuren stehen gleiche Bezugszahlen für gleiche oder ähnliche Bauteile.

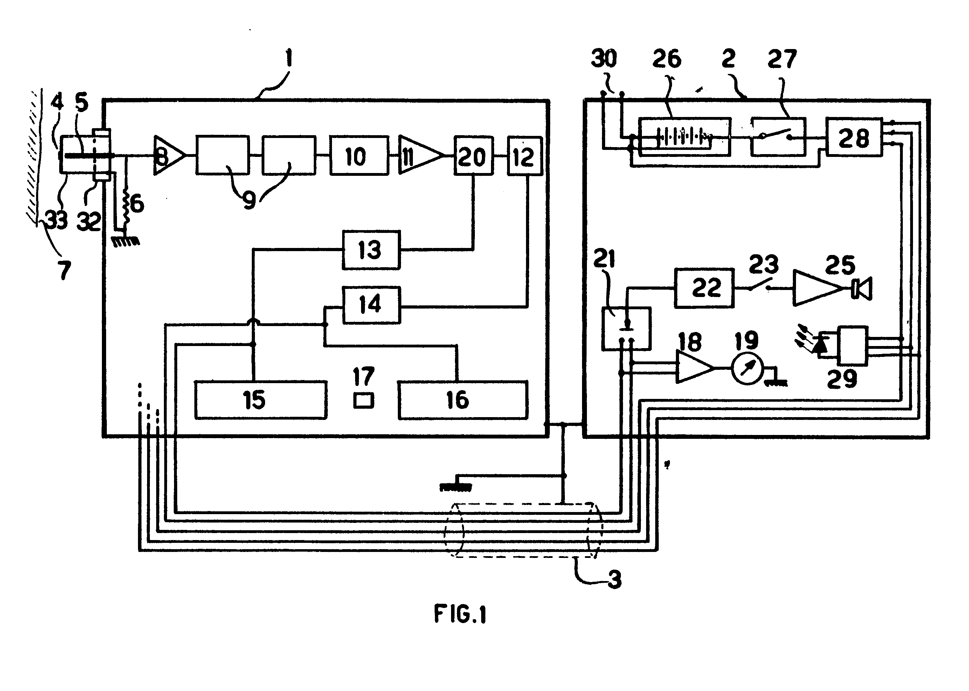

Gemäss Figur 1 besteht die Vorrichtung aus einem in einem getrennten Gehäuse untergebrachten Teil 1 und aus einem zweiten Teil 2, der ebenfalls in einem getrennten Gehäuse untergebracht ist und mit dem ersten Teil 1 durch ein flexibles, abgeschirmtes Kabel 3 verbunden ist, wobei der erste Teil die Sonde, Filterstufen, Verstärkung und Anzeige umfasst und der zweite Teil die Messchaltung und Energieversorgung enthält.

Anstatt die beiden Teile 1 und 2 in separaten Gehäusen unterzubringen ist es auch möglich die beiden Teile in einem Gehäuse zu vereinigen.

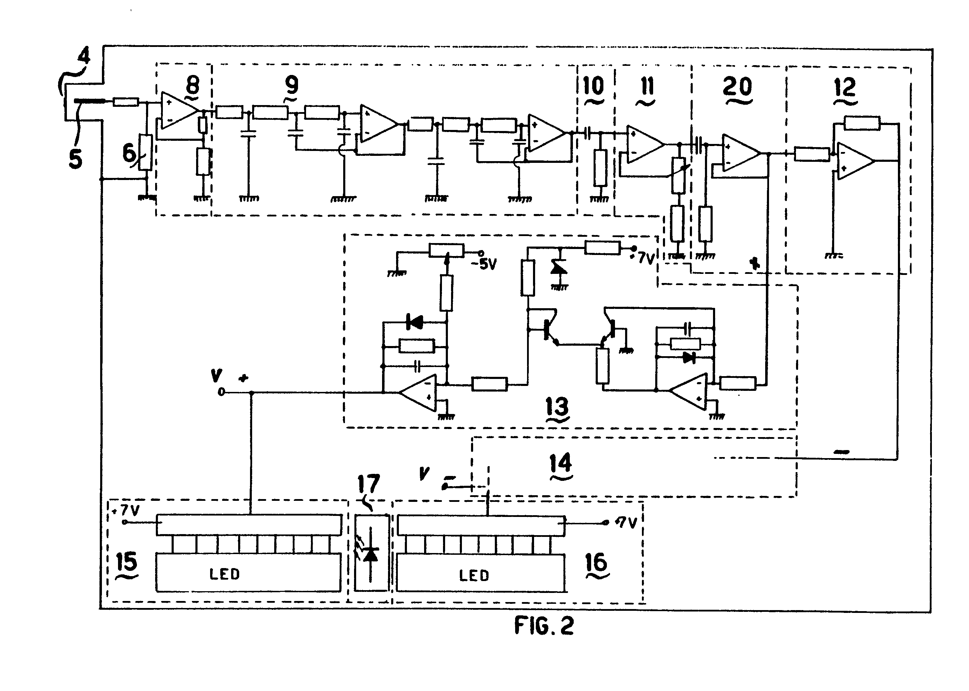

Nach der in den Figuren 1 und 2 dargestellten Ausführungsform besteht die Sonde aus einer äusseren Abschirmung und einer Messelektrode 5 aus elektrisch leitendem Material, die von der Abschirmung und vom Gehäuse isoliert ist.

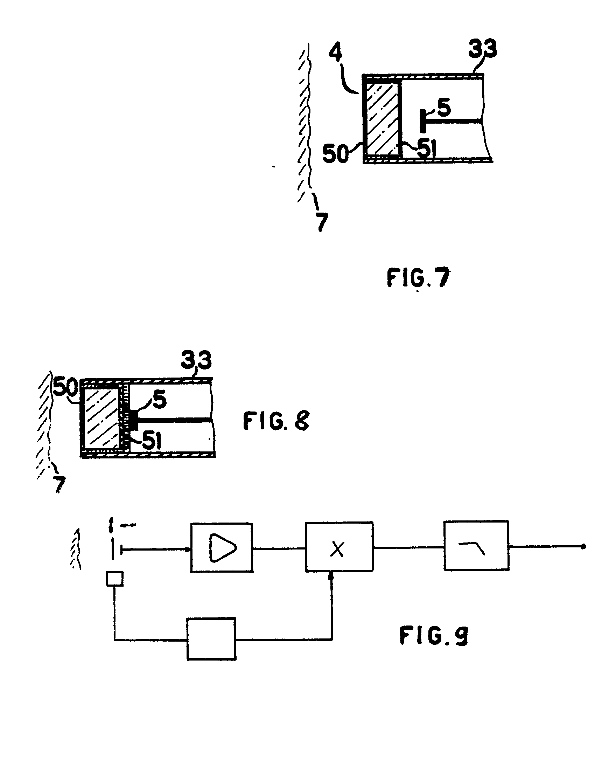

Gemäss einer Ausführungsform ist die Messelektrode 5 an einer Seite in einer Wandungsöffnung eingelassen, wobei diese Wandung 32 am Gehäuse befestigt ist und von ihm elektrisch isoliert ist und wobei eine metallische Verkleidung 33 als Abschirmung dienend die Messelektrode bis in die Wandung hinein umhüllt und das freie Ende der Messelektrode durch ein Fenster 4, das parallel zur Organismusoberfläche ausrichtbar ist, dem elektrostatischen Feld zugänglich lässt. Das Fenster 4 wird erfindungsgemäß, wie in den Figuren 7 und 8 dargestellt ist, aus zwischen zwei leitenden Schichten eingefügtes polarisierbares Flüssigkristall gebildet.

Es können ebenfalls nicht leitende Materialien, die eine höhere dielektrische Konstante besitzen als die Luft, auf dem Fenster 4 angebracht werden, um die Kapazität des durch die Organismusoberfläche und durch das freie Elektrodenende gebildeten Kondensators zu erhöhen.

Die Sonde wird so nahe an der Organismusoberfläche 7 gehalten, dass die Ladung an der Messelektrode 5 sich der Ladung auf der Oberfläche 7 in der Grösse anpasst, aber von entgegengesetztem Zeichen. Dadurch werden Elektronen heran oder zur Erde weggeleitet. So fliesst also ein Strom durch den Ausgleichwiderstand 6. Da der Widerstandswert sehr hoch ist, genügt ein geringer Strom um eine Spannung aufzubauen, die von einem Verstärker 8 aufgenommen werden kann.

Die Wandung 32 ist so ausgebildet, dass Messelektrode 5 und Verkleidung 32 leicht ausgewechselt und den gegebenen Umständen angepasst werden können, wie z. B. mittels Schraubstopfen, Bajonettfassung, Klemmung, usw.

Die gemessenen Signale haben eine Frequenz von 0 bis 10 Hz; die höheren Frequenzen werden durch Filter 9 mit 36 dB/Okt abgeschnitten. Dadurch werden störende elektrische Signale ausgefiltert.

Um den Messausschlag der Vorrichtung unabhängig von in den Verstärkerstufen hervorgerufenen Offsetspannungen zu halten, werden Hochdurchlassfilter 10 und 20 angebracht.

Nach Figur 2 kann diese Filtereigenschaft durch zwei Differentialschaltungen 10 und 20 erreicht werden.

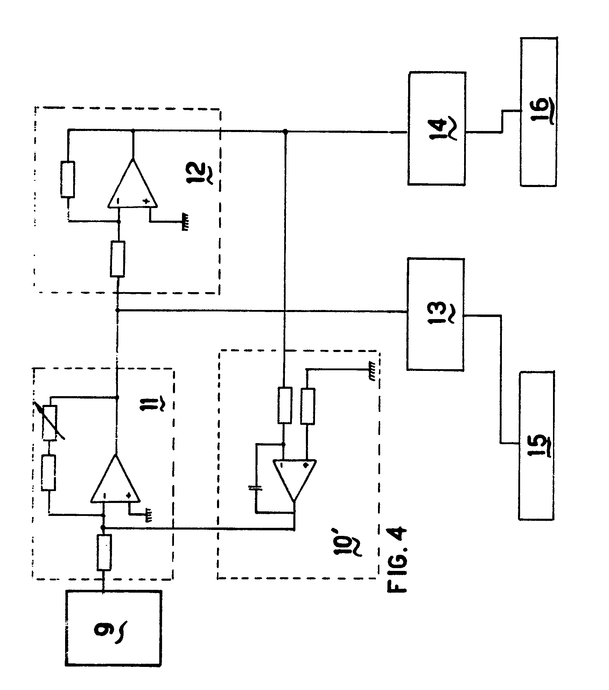

Gemäss Figur 4 kann das gleiche Resultat mit einer Integrationsschaltung in Gegenkopplung 10' erreicht werden, wobei jedoch im Gesamten weniger Bauelemente benutzt werden.

Das Signal wird durch den Verstärker 11 zuerst verstärkt und zur weiteren Verarbeitung in ein positives und ein negatives Signal getrennt. Beide Signale werden logarithmisch verstärkt, sodass ein schwaches Signal um 50 dB mehr verstärkt wird als ein starkes Signal. Das positive Signal wird im Verstärker 13 logarithmisch verstärkt und das negative Signal im Verstärker 14. Leuchtdioden (L.e.d.) 15 und 16 zeigen die Signale diskret an. Der Nullstand wird laufend durch eine zusätzliche Diode 17 zur Orientierung angegeben.

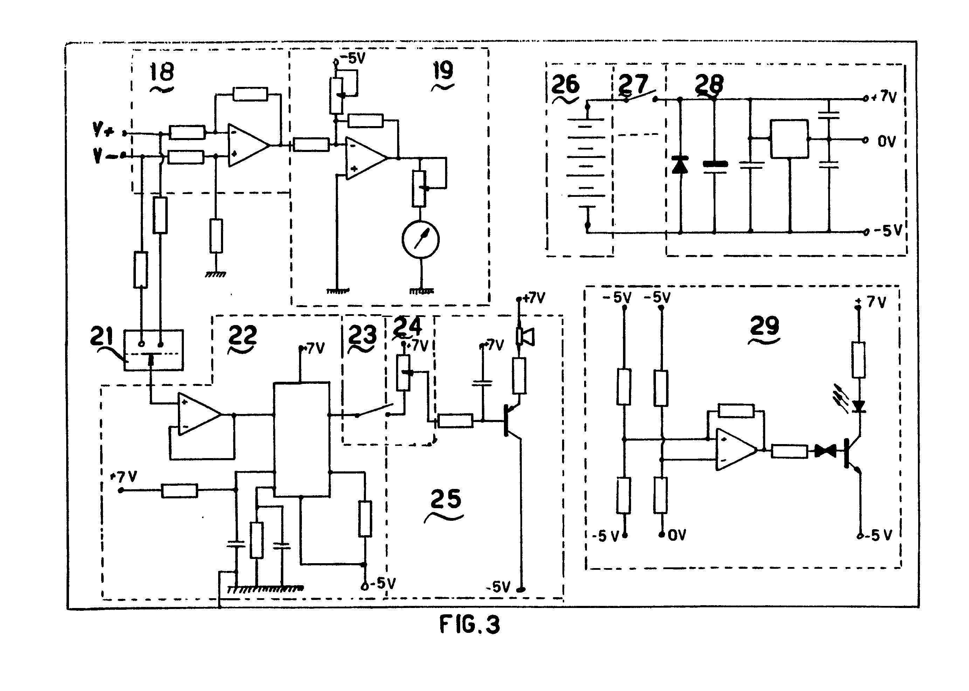

Gemäss der unter Bezug auf die Figuren 1, 7 und 8 erläuterten Ausführungsform der Erfindung enthält Teil 2 der Vorrichtung ein analoges mA-Meter 19, dessen Signal von einem Differentialverstärker 18 kommt. Figur 3 gibt die Detailschaltung dieser Funktionen.

Man kann zusätzlich auch ein akustisches Signal hervorrufen, das mittels Schalter 21 entweder das positive Signal, das negative Signal oder beide Signale wiedergibt. Die akustische Wiedergabe 25 kann durch einen Schalter 23 abgeschaltet werden. Das akustische Signal erübrigt die permanente Überwachung der Anzeigevorrichtung.

Nach Figur 3 kann diese akustische Wiedergabe durch einen Umwandler 22 erreicht werden, der Spannungen in Frequenzen umsetzt, sodass die Frequenz ansteigt sobald die Spannung zunimmt. Man kann ebenfalls eine Lautstärkeregelung durch ein Potentiometer 24 vorsehen.

Der zweite Teil der Vorrichtung weist eine Energieversorgungsquelle, auf die vorzugsweise aus einem 12 V-Akkulader 26 beseht oder aus einer Batterie. Durch Schalter 27 kann die gesamte Vorrichtung unter Spannung gesetzt werden. Man kann auch einen Gleichrichter 28 vorsehen, der die schwankenden Batteriespannungen zwischen 10 und 12 V stabilisiert.

Eine Ladekontroll-Leuchte 29 kann ebenfalls eingebaut werden.

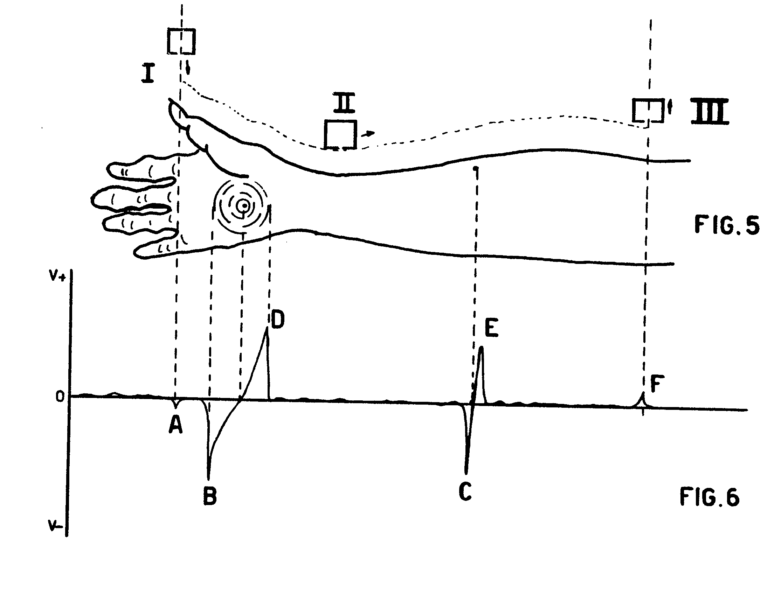

Anhand der Figuren 5 und 6 wird nun eine Anwendung der erfindungsgemässen Vorrichtung bei der Abtastung eines Vorderarmes erklärt.

Bei Position I wird die Sonde der Organismusoberfläche genähert. Sobald die Sonde nahe genug ist, vorzugsweise etwa 10 cm, fliesst ein erster Strom durch den hochohmigen Widerstand um die automatische Nulleinstellung zu erreichen (Position A in Figur 6).

Bei Position II wird die Band auf Abstand abgetastet. Sobald man sich einem Punkt nähert, in dem ein pathologischer Prozess abläuft, kann ein typischer Spannungsverlauf B-D oder C-E festgestellt werden.

In Position III wird die Sonde zurückgezogen und die Anzeigevorrichtung schlägt in entgegengesetztem Sinn im Vergleich zu A aus, da die Kapazität des Kondensators durch die immer grösser werdende Distanz verändert wird.

Der Anschlag der Anzeigevorrichtung bei pathologischen Punkten ist deutlich vom restlichen Kurvenverlauf trennbar.

Das Spannungsdiagramm gemäss Figur 6 lässt zwischen B und D einerseits und C und E andererseits deutlich zwei Zonen erkennen in welchen messbare Spannungsunterschiede. herrschen. In der breiteren Zone zwischen B und D wird eine Anomalie festgestellt, in welcher sich ein pathologischer Prozess abwickelt. Dabei kann es sich um einen Genesungsprozess, wie zum Beispiel das Heilen einer Wunde oder ein Schmerzzentrum, z. B. ein Tumor handeln.

Der Spannungsverlauf zwischen C und E deutet auf eine konzentrierte aktieve Zone hin. Hier handelt es sich um einen sogenannten Akupunkturpunkt.

Das Spannungsdiagramm der Figur 6 welches die Aufzeichnung einer Messung darstellt die mit einer nicht unter Anspruch 1 fallenden Vorrichtung erhalten wurde, lässt gleich zwei wichtige medizinische Anwendungsmöglichkeiten erkennen.

Die erste Anwendung ist, wie es die Kurve zwischen C und D verdeutlicht, das präzise Aufsuchen von Akupunkturpunkten.

Die zweite Anwendungsmöglichkeit ist das Aufsuchen eines pathologischen Prozesses, wie z. B. eines schwer lokalisierbaren Schmerzzentrums. Es kommt nämlich sehr oft vor, dass eine allgemeine Schmerzempfindung keine präzise Aussage über das Zentrum des Schmerzes und folglich für den Arzt keine Feststellung der Ursache des Schmerzen bzw. Diagnostik zulässt. Diese Tatsache gewinnt besonders an Wert bei Tieren oder sogar Pflanzen welche nicht in der Lage sind, eine Auskunft betreffend ein Schmerzzentrum zu geben.

Das Spannungsdiagramm aus Figur 6 lässt ebenfalls bei A und F erkennen, dass eine Bewegung senkrecht zur abzutastenden Oberfläche ein Messignal auslöst. Daraus könnte man schliessen, dass zur Vermeidung von Störsignalen dem Messverfahren die Bedingung vorausgesetzt ist, dass die Sonde parallel zu den Messoberflächen, d.h. unter Beibehalten eines konstanten Abstandes, geführt werden muss. Figur 6 zeigt jedoch, dass die Amplituden der Signale A und F wesentlich kleiner sind als diejenigen der Signal B-D und C-E, d.h. dass eine Abweichung von der optimalen Tastbewegung zwar ein Störsignal auslöst, aber dass die Überlagerung eines solchen Störsignals wegen seiner geringen Amplitude, mit einem Messignal A-D oder C-E keinen störenden Einfluss auf letztere hat, d.h. auf Grund ihrer Amplituden kann man sehr leicht die Messignale von den Störsignalen unterscheiden.

Ein wesentliches Merkmal des anhand der Figuren 5 und 6 erläuterten Verfahrens, das unter Verwendung der in den Figuren 1 bis 4 und 7, 8 beschriebenen Vorrichtung durchgeführt werden kann, besteht darin, dass keine Absolutwerte, sondern Potential- oder Ladungsgradienten gemessen werden. Dadurch werden störend wirkende Faktoren, wie z. B. statische Elektrizität auf der Messoberfläche, Kleidung, Bandagen, Tierfell, usw. automatisch eliminiert.

Nach der vorliegenden Erfindung wird die dielektrische Konstante des aus Flüssigkristallen bestehenden Fensters 4 moduliert. Dieses Material weist die Besonderheit auf, bei verschiedener Polarisation eine verschiedene dielektrische Konstante zu besitzen. Nach Figur 7 besteht demnach die Sonde aus der Messelektrode 5, der Abschirmung 33 und dem Fenster 4, das aus Flüssigkristallen besteht, die durch eine Modulationsspannung an den elektrisch leitenden Schichten 50 und 51 polarisiert werden, wobei die leitenden Schichten von der Abschirmung elektrisch isoliert sind.

Figur 8 zeigt eine bevorzugte, sehr kompakte Ausführungsform, in der die Messelektrode 5 isoliert auf dem Flüssigkristallfenster 4 befestigt ist.

Nach dem Blockschaltbild der Figur 9 kann das gemessene Signal synchron mit einem Referenzsignal verstärkt werden. Dieses Referenzsignal kann, z. B. von einem Oszillator, der gleichzeitig die Feldmodulation anregt stammen.

## Patentansprüche

1. Vorrichtung für das kontaktlose Messen von Ladungskonzentrationen und Potentialunterschieden von biologischen Organismen mit einer abgeschirmten elektrisch leitenden und feststehenden Messelektrode (5), die durch ein in der Abschirmung (33) geschaffenes Fenster (4) für ein elektrisches Feld zugänglich ist, mit einer Schaltung zur elektrischen Verbindung der leitenden Messelektrode (5) mit einem Referenzpotential, sowie mit einer Schaltung zum Messen und Anzeigen (15, 16, 17, 19) des aufgefangenen Feldes und einer Energieversorgungsquelle (26), dadurch gekennzeichnet, dass die dielektrische Konstante zwischen der Organismusoberfläche (7) und der Messelektrode durch zwischen zwei leitende Schichten (50, 51) eingefügtes polarisierbares Flüssigkristall veränderbar ist, dass an die Schichten (50, 51) eine Wechselspannung angelegt ist und dass dieses Flüssigkristall im Fenster (4) zwischen Organismusoberfläche (7) und Messelektrode (5) angeordnet ist.

2. Vorrichtung nach Anspruch 1, dadurch gekennzeichnet, dass das verstärkte Messignal in ein positives und ein negatives Signal aufgeteilt wird und dass beide Signale logarithmisch verstärkt werden, so daß bei einer Bewegung der Messelektrode entlang der Organismusoberfläche eine Andeutung eines Messpunktes erhältlich ist.

## Claims

1. Device for the contactless measuring of charge concentrations and potential differences of biological organisms, comprising a shielded electrically conductive and fixed measuring electrode (5) which is accessible to an electric field through a window (4) created in the shielding (33), including a circuit for electrically connecting the conductive measuring electrode (5) to a reference potential and including a circuit for measuring and displaying (15, 16, 17, 19) the captured field, and a power supply source (26), characterized in that the dielectric constant between the organism surface (7) and the measuring electrode can be changed by a polarizable liquid crystal inserted between two conductive layers (50, 51), that an alternating voltage is applied across the layers (50, 51) and that this liquid crystal is arranged in the window (4) between organism surface (7) and measuring electrode (5).

2. Device according to Claim 1, characterized in that the amplified measurement signal is divided into a positive and a negative signal and that both signals are logarithmically amplified so that an indication of a measurement point can be obtained when the measuring electrode is moved along the organism surface.

## Revendications

1. Dispositif pour la mesure, sans contact, de concentrations de charges et de différences de potentiel dans des organismes biologiques, ce dispositif comportant une électrode de mesure 5 qui est fixe, conductrice de l'électricité et blindée et qui est accessible pour un champ électrique via une fenêtre (4) pratiquée dans le blindage (33), ce dispositif comportant également un circuit pour le raccordement électrique de l'électrode de mesure conductrice (5) à un potentiel de référence, de même qu'un circuit pour la mesure et l'affichage (15, 16, 17, 19) du champ capté, ainsi qu'une source d'alimentation d'énergie (26), caractérisé en ce que la constante diélectrique peut être modifiée entre la surface (7) de l'organisme et l'électrode de mesure par un cristal liquide polarisable introduit entre deux couches conductrices (50, 51), une tension alternative étant appliquée aux couches (50, 51), tandis que ce cristal liquide situé dans la fenêtre (4) est disposé entre la surface (7) de l'organisme et l'électrode de mesure (5).

2. Dispositif selon la revendication 1 caractérisé en ce que le signal de mesure amplifié est divisé en un signal positif et en un signal négatif, tadis que les deux signaux font l'objet d'une amplification logarithmique de telle sorte que, lors d'un mouvement de l'électrode de mesure le long de la surface de l'organisme, on obtienne une indication d'un point de mesure.

**FIG.1**

FIG. 2

FIG. 3

FIG. 4

I II III

FIG.5

FIG.6

V+

0

V-

A B C D E F

FIG.7

FIG. 8

FIG.9